## Europäisches Patentamt
## European Patent Office
## Office européen des brevets

(19)

(11) Numéro de publication: **0 148 653**
**B1**

(12)

# FASCICULE DE BREVET EUROPEEN

(45) Date de publication du fascicule du brevet:
**16.09.87**

(21) Numéro de dépôt: **84402235.0**

(22) Date de dépôt: **07.11.84**

(51) Int. Cl.⁴: **A 61 B 17/22**

(54) Appareil à impulsions ultrasonores destiné à la destruction des calculs.

(30) Priorité: **14.12.83 FR 8320041**

(43) Date de publication de la demande:
**17.07.85 Bulletin 85/29**

(45) Mention de la délivrance du brevet:
**16.09.87 Bulletin 87/38**

(84) Etats contractants désignés:
**BE CH DE GB IT LI NL SE**

(56) Documents cités:
**DE - A - 2 722 252**
**DE - A - 3 119 295**
**DE - A - 3 122 056**
**FR - A - 2 487 664**

**ULTRASONICS, vol. 5, avril 1967, pages 105-112; P.P. LELE: "Production of deep focal lesions by focused ultrasound-current status**

(73) Titulaire: **Dory, Jacques, 91 rue des Molveaux, F-77450 Coupvray Esblay (FR)**

(72) Inventeur: **Dory, Jacques, 91 rue des Molveaux, F-77450 Coupvray Esblay (FR)**

(74) Mandataire: **Marquer, Francis et al, CABINET MOUTARD 35, avenue Victor Hugo Résidence Champfleury, F-78180 Voisins-le-Bretonneux (FR)**

# Description

L'invention concerne un lithotripteur réalisable à un coût moins élevé que ceux qui font appel à la génération d'ondes de choc au moyen d'un éclateur et au repérage du calcul par radioscopie et présentant, par rapport à ces appareils connus, différents avantages substantiels, en particulier l'obtention d'une localisation plus précise et plus sûre du calcul et une mise en oeuvre simplifiée.

Dans un appareil connu, décrit dans le document DE-A-2 722 252, des ondes de choc sont engendrées par décharges électriques effectuées à un foyer d'un réflecteur elliptique et se trouvent focalisées au second foyer de l'ellipse. L'énergie de la tache focale ne sera suffisante pour détruire un calcul intra-rénal (des pressions de l'ordre de $10^8$ Pascal sont nécessaires) que si la transmission se fait dans un liquide (eau) parfaitement dégazé, ce qui est une contrainte d'utilisation importante. Ce procédé requiert par ailleurs le changement d'électrodes onéreuses de l'éclateur à chaque traitement ou même en cours de traitement. La tache focale produite est relativement grosse. Enfin le repérage de la position de la région focale s'effectue au moyen d'un dispositif auxiliaire d'échographie dont le ou les transducteurs sont disposés, nécessairement, à l'écart de l'axe du réflecteur elliptique.

Par ailleurs, le document DE-A-3 119 295 décrit un lithotripteur du type défini dans le préambule des revendications indépendantes 1 et 2. Pour le repérage de la position de la région focale, ce document suggère soit de mettre en oeuvre un dispositif auxiliaire d'échographie tel que décrit dans le document DE-A-2 722 252 précité, soit d'utiliser le transducteur principal lui-même pour former une partie du dispositif auxiliaire d'échographie.

L'invention se propose d'améliorer ces dispositifs connus, de la façon définie dans la partie caractérisante des revendications indépendantes 1 et 2, respectivement. Dans les deux cas, elle propose de mettre en oeuvre un transducteur auxiliaire, distinct du transducteur principal en coupelle sphérique, mais solidarisé à ce dernier et émettant pendant les intervalles entre les impulsions principales.

La revendication 1 définit en outre une disposition particulièrement adaptée du transducteur auxiliaire suivant l'axe de la coupelle sphérique. Ainsi, au cours du déplacement du transducteur principal qui permet d'amener la tache focale dans la région désirée du calcul, le transducteur auxiliaire fournit ainsi à tout moment une image du calcul et de la tache focale, ce qui permet d'effectuer facilement et avec précision la localisation désirée.

Suivant une autre particularité de l'invention définie dans la revendication 2, l'appareil comporte notamment des moyens de faire émettre au transducteur principal, pendant les intervalles entre les impulsions principales, des impulsions de contrôle ayant une puissance substantiellement réduite, à la même cadence que les impulsions émises normalement par le transducteur auxiliaire qui n'émet alors pas lui-même, les échos correspondants reçus par le transducteur auxiliaire pouvant être traités pour contrôler la localisation et la répartition de l'énergie des impulsions principales.

D'autres particularités, ainsi que les avantages de l'invention, apparaîtront clairement à la lumière de la description ci-après.

Au dessin annexé:

la figure 1 est le schéma de principe d'un appareil conforme à un mode d'exécution préféré de l'invention;

la figure 2 représente schématiquement, en perspective, le transducteur principal et son dispositif de support mobile;

la figure 3 représente les formes d'ondes en différents points des circuits de l'appareil; et

la figure 4 illustre l'image obtenue sur l'écran de visualisation que comporte l'appareil.

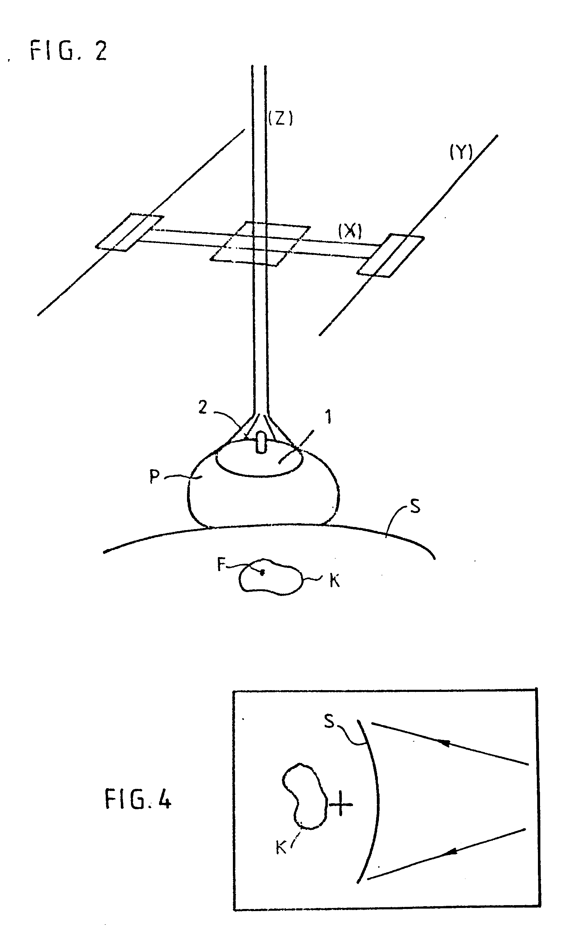

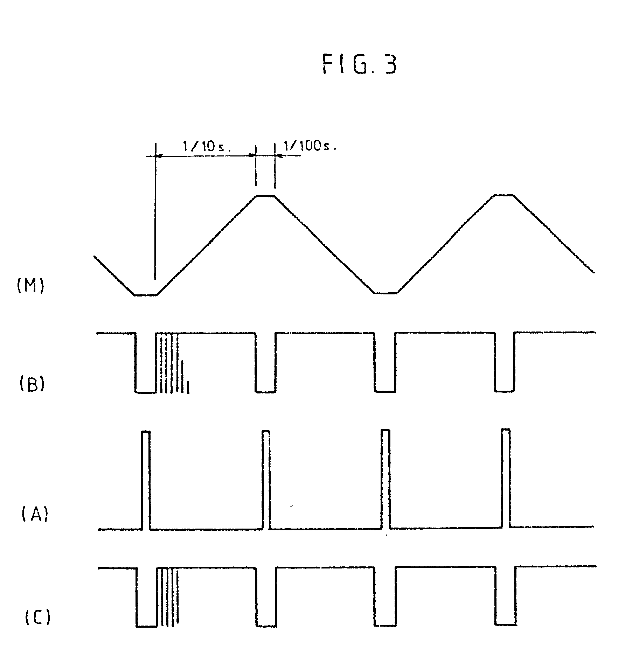

A la figure 2, on a représenté un transducteur 1 en forme de calotte sphérique supporté par un montage qui en permet les déplacements suivant trois axes orthogonaux X, Y et Z. Ce montage a été représenté de manière schématique, sa réalisation étant à la portée de l'homme du métier. Suivant l'axe de la calotte sphérique est disposé un transducteur auxiliaire 2 de forme générale cylindrique, qui passe à travers la calotte 1 et lui est fixé. Une poche d'eau P est interposée entre la calotte 1 et la surface S du corps du patient, celui-ci étant supposé allongé sur un plan horizontal.

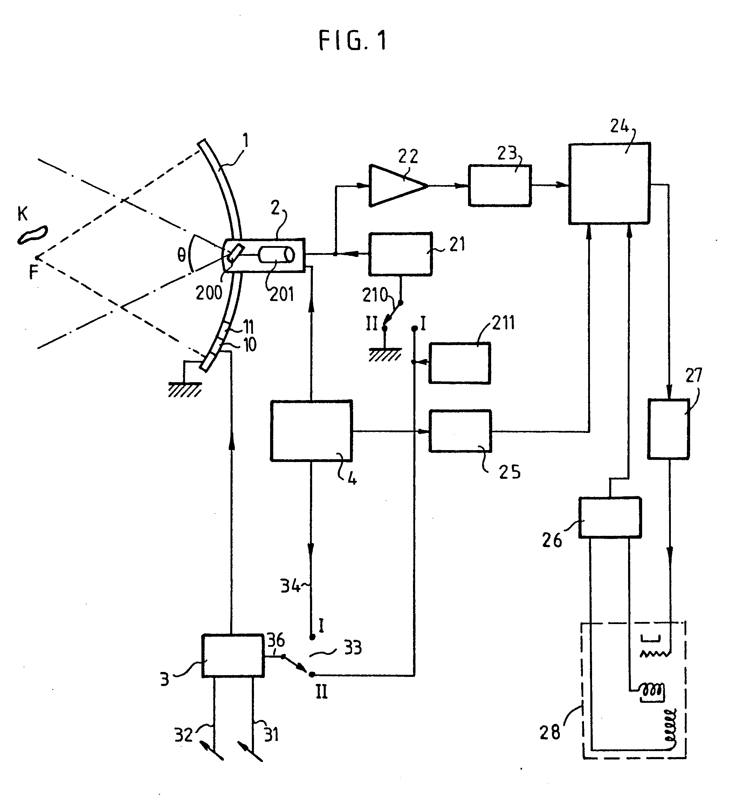

La calotte 1 a par exemple 200 à 300 mm de diamètre et est composée d'un grand nombre (300 ou 400) d'éléments piézo-électriques 10, 11, etc... (figure 1) isolés les uns des autres et juxtaposés pour constituer une mosaïque. Ces éléments sont métallisés sur leurs deux faces, l'une des métallisations étant reliée à la masse et l'autre à des connexions d'excitation par un émetteur 3.

Ce dernier fournit un signal électrique A (figure 3) composé de paquets d'impulsions à haute fréquence (500 KHz par exemple) de brève durée (par exemple 1 µS). Chaque paquet comprendra par exemple un nombre d'impulsions compris entre 1 et 10 et ces impulsions correspondent à une puissance de crête très élevée (de l'ordre de 100 Kw par exemple).

Une telle puissance peut être obtenue au moyen d'un émetteur d'impulsions faisant appel à des technologies bien connues, soit avec des transistors de puissance, soit avec des thyristors. Il pourra être envisagé d'exciter tous les éléments en parallèle, ou d'exciter des groupes d'éléments mis en série.

On a symbolisé par une entrée 31 de l'émetteur 3 un réglage de la puissance émise et par une entrée 32 un réglage de la forme d'impulsion. Ces réglages permettent de contrôler la forme du signal produit au niveau de la tache focale, au centre F de la sphère. Celle-ci pourra, avec cette technique, être très petite (diamètre de 2 ou 3 mm par exemple) et avoir une position rigoureusement fixe pour une position donnée du transducteur.

A la figure 1, on voit que le transducteur auxiliaire 2 est lui-même relié, d'une part, à un émetteur d'impulsions électriques 21, d'autre part, à un amplificateur de réception 22 suivi d'un convertisseur analogique-numérique 23 lui-même suivi d'une mémoire 24. L'émetteur 21 est synchronisé par un générateur d'impulsions 211 qui fournit 256 impulsions (créneau B, figure 3) en une durée (1/10 sec. par exemple) inférieure à la période des impulsions A. Cette durée correspond au temps de balayage complet d'un secteur angulaire prédéterminé θ (figure 1) par le faisceau émis par le transducteur 2, donc de formation, dans le plan de balayage, d'une image du calcul K.

Le transducteur 2 est avantageusement du type décrit dans les demandes de brevet français FR-A-2 487 664 déposée le 29 Juillet 1980 pour: Sonde d'échographie à balayage sectoriel comportant deux liquides de couplage et FR-A-2 487 665 déposée le 29 Juillet 1980 pour: Sonde d'échographie à balayage sectoriel mécanique, c'est-à-dire qu'il comporte un élément piézoélectrique oscillant 200 commandé par un moteur 201, lui-même commandé par un circuit électronique que l'on a symbolisé par un rectangle 4. Ce circuit électronique fournit des signaux (M, figure 3) de commande du moteur 201 logé à l'intérieur du boîtier du transducteur 2 et est agencé de façon qu'une oscillation complète du moteur corresponde à la durée de formation d'une image définie ci-dessus (1/10 sec.). Un court intervalle de temps prédéterminé après la fin du créneau B, le commutateur 33 étant supposé en position I, le circuit 4 envoie une impulsion de 1/100 sec. par exemple, transmise à l'entrée 36 de l'émetteur 3, de façon à déclencher l'émission d'un paquet d'impulsions A.

Pendant cette impulsion de déclenchement, l'élément oscillant 200 reste immobile en fin de balayage, si bien que la sonde ne reçoit pas d'échos correspondants.

Il est possible de supprimer la liaison entre le générateur 211 et l'émetteur 21 en mettant le commutateur 210 en position II. Dans ce mode de fonctionnement, l'émetteur 21 n'est plus en service et le transducteur 2 n'est plus utilisé qu'en réception. Par contre, le commutateur 33 étant supposé en position II en même temps que le commutateur 210 (le couplage entre ces deux commutateurs n'a pas été figuré), l'émetteur 3 est alors synchronisé par le générateur 211, si bien qu'il émet des impulsions C (figure 3) à la même cadence que les impulsions B mentionnées précédemment.

L'émetteur 3 excite ainsi, lorsque les commutateurs sont en position II, le transducteur 1 avec un signal C composé de séquences de 256 impulsions par exemple. Les échos correspondants sont reçus par le transducteur 2, si bien qu'une image de la zone de concentration de l'énergie alors émise par le transducteur 1 est obtenue, comme on l'expliquera plus loin.

Les impulsions B émises par l'émetteur 21 et les impulsions C émises par l'émetteur 3 sont de faible puissance (quelques watts de crête). La puissance de l'émetteur 21 est réglable et celle de l'émetteur 3 est, au moment de la commutation du commutateur 33 vers la position II, considérablement réduite, par exemple par une réduction importante de la tension d'alimentation de l'émetteur 3, réduction commandée par ladite commutation de façon connue en soi et non figurée.

Les signaux reçus en 22, quelle que soit leur nature, sont, après conversion analogique-numérique en 23, stockés ligne par ligne dans la mémoire 24, un dispositif d'adressage d'écriture 25, commandé par le circuit 4, permettant de faire correspondre les angles respectifs de déviation du faisceau émis et/ou reçu par le transducteur 2 aux lignes respectives de la mémoire. Un dispositif 26 de lecture rapide de la mémoire excite les bobines de déviation en X et en Y d'un tube cathodique 28, donc l'électrode de commande de brillance reçoit le contenu correspondant de la mémoire 24, transformé en signal analogique par un convertisseur numérique-analogique 27.

La réalisation pratique de tous les circuits décrits et représentés est à la portée de l'homme de l'Art.

L'appareil qui vient d'être décrit fonctionne de la manière suivante:

Comme on l'a indiqué ci-dessus, en fonctionnement normal, les commutateurs sont en position I et des impulsions ultrasonores correspondant aux impulsions A sont engendrées par le transducteur 1 et focalisées au centre F de la sphère. Pendant la durée de chaque créneau B, le dispositif d'échographie à balayage sectoriel constitué par le transducteur, l'émetteur auxiliaire et les organes de réception, de traitement et de visualisation 22 à 27, affiche sur l'écran une image de la zone balayée, donc du rein et du calcul K.

Par ailleur, le dispositif de visualisation est agencé, de manière connue en soi, pour matérialiser sur l'écran du tube cathodique (par exemple par une croix) la position théorique de la tache focale dans le plan de coupe représenté, plan qui passe par l'axe de symétrie du transducteur 1. (Il s'agit d'échographie du type B). L'opérateur commence par déplacer le transducteur 1 en X, jusqu'à ce que le calcul apparaisse nettement sur l'écran, puis il le déplace en Y et Z, jusqu'à ce que la croix coïncide avec la région centrale de l'image du calcul. A ce moment, les commutateurs peuvent être mis en position II; la région de la tache focale est alors rendue visible sur l'écran, avec une luminosité proportionnelle à la concentration d'énergie correspondante. On a ainsi une représentation de ce que sera la répartition de l'énergie de l'onde de choc pendant le tir, ce qui permet de contrôler et de parfaire les réglages.

Il est clair que l'appareil décrit permet le contrôle de l'évolution du calcul après chaque tir. Il va de soi qu'il pourra subir diverses modifications et même faire l'objet de variantes d'exécution, sans s'écarter de la portée de l'invention telle que définie dans les revendications jointes.

## Revendications

1. Lithotripteur comprenant des moyens d'engendrer des ondes de choc concentrées en une région focale et des moyens de repérage de la position de ladite région focale, lesdits moyens générateurs comprenant un générateur d'impulsions ultrasonores (1-3) comportant un transducteur piézoélectrique principal (1) dont la surface active est une coupelle sphérique, lesdits moyens de repérage comportant un dispositif d'échographie comprenant un générateur d'impulsions auxiliaires associé à un transducteur auxiliaire (2), caractérisé en ce que ledit transducteur auxiliaire est solidarisé à ladite coupelle sphérique (1) et disposé suivant l'axe de celle-ci, qu'il émet pendant les intervalles entre les impulsions principales, et que le dispositif d'échographie auxiliaire est du type à balayage sectoriel effectué de manière à former l'image pendant lesdits intervalles dans un plan de symétrie de ladite coupelle sphérique.

2. Lithotripteur comprenant des moyens d'engendrer des ondes de choc concentrées en une région focale et des moyens de repérage de la position de ladite région focale, lesdits moyens générateurs comprenant un générateur d'impulsions ultrasonores (1-3) comportant un transducteur piézoélectrique principal (1) dont la surface active est une coupelle sphérique, lesdits moyens de repérage comportant un dispositif d'échographie comprenant un générateur d'impulsions auxiliaires associé à un transducteur auxiliaire (2), caractérisé en ce que ledit transducteur auxiliaire est solidarisé à ladite coupelle sphérique (1), qu'il émet pendant les intervalles entre les impulsions principales, et qu'il est prévu des moyens (210, 211, 33, 3) pour interrompre l'émission d'impulsions auxiliaires par le transducteur auxiliaire et faire émettre audit transducteur principal (1), pendant les intervalles entre les impulsions principales (A), une pluralité d'impulsions (C) de contrôle de la tache focale, ayant une puissance sensiblement plus réduite que celle des impulsions principales, à la même cadence que celles (B) émises normalement par le transducteur auxiliaire (2).


## Patentansprüche

1. Lithotripter mit Mitteln zur Erzeugung von Schockwellen, die in einem Fokalbereich konzentriert werden und Mitteln zur Ortung des besagten Fokalbereiches, wobei besagte Erzeugungsmittel einen Ultraschallimpulsgenerator (1-3) enthalten, mit einem piezoelektrischen Hauptwandler (1), dessen aktive Oberfläche eine kugelförmige Schale ist, und besagte Ortungsmittel eine Echographievorrichtung mit einem Hilfsimpulsgenerator enthalten, der einem Hilfswandler (2) zugeordnet ist, dadurch gekennzeichnet, dass besagter Hilfswandler fest mit der besagten kugelförmigen Schale (1) verbunden und entlang deren Achse angeordnet ist und während der Zeiträume zwischen den Hauptimpulsen sendet und dass die Hilfsechographievorrichtung so ausgelegt ist, dass sie eine abschnittweise Abtastung vornimmt, um so während besagter Zwischenzeiten das Bild in einer Symmetrieebene der besagten kugelförmigen Schale zu formen.

2. Lithotripter, mit Mitteln zur Erzeugung von Schockwellen, die in einem Fokalbereich konzentriert werden und Mitteln zur Ortung des besagten Fokalbereiches, wobei besagte Erzeugungsmittel einen Ultraschallimpulsgenerator (1-3) enthalten, mit einem piezoelektrischen Hauptwandler (1), dessen aktive Oberfläche eine kugelförmige Schale ist und besagte Ortungsmittel eine Echographievorrichtung enthalten, mit einem Hilfsimpulsgenerator, der einem Hilfswandler (2) zugeordnet ist, dadurch gekennzeichnet, dass besagter Hilfsgenerator fest mit besagter kugelförmiger Schale (1) verbunden ist und während der Zeiträume zwischen den Hauptimpulsen sendet und dass Mittel (210, 211, 33, 3) vorgesehen sind, um die Abgabe von Hilfsimpulsen durch den Hilfswandler zu unterbrechen und den besagten Hauptwandler (1) zu veranlassen, zwischen den Hauptimpulsen (A) eine Vielzahl von Impulsen (C) zur Kontrolle der Fokalstelle auszusenden, deren Stärke wesentlich geringer ist, als die der Hauptimpulse, in der gleichen zeitlichen Abfolge wie die (B), welche normalerweise vom Hilfswandler (2) abgegeben werden.


## Claims

1. A lithotrite comprising means for generating shock waves concentrated in a focal region and means for locating the position of said focal region, said generating means comprising an ultrasonic pulse generator (1-3) comprising a main piezoelectric transducer (1) whose active surface is a spherical cap, said locating means comprising an echography device including an auxiliary pulse generator associated to an auxiliary transducer (2), characterized in that said auxiliary transducer is fixed to said spherical cap (1) and disposed along the axis thereof, and emits during the intervals between the main pulses, and in that the auxiliary echography device is of the type with sectorial sweep effected so as to form an image during said intervals in a plane of symmetry of said spherical cap.

2. A lithotrite comprising means for generating shock waves concentrated in a focal region and means for locating the position of said focal region, said generating means comprising an ultrasonic pulse generator (1-3) comprising a main piezoelectric transducer (1) whose active surface is a spherical cap, said locating means comprising an echography device including an auxiliary pulse generator associated to an auxiliary transducer (2), characterized in that said auxiliary transducer is fixed to said spherical cap (1), in that it emits during the intervals between the main pulse and in that means (210, 211, 3) are provided for interrupting the emission of auxiliary pulses by the auxiliary transducer and having said main transducer (1) emit during the intervals between the main pulses (A), a plurality of control pulses (C) of the focal spot, having a power substantially more reduced that that of the main pulses, at the same rate as those (B) normally emitted by the auxiliary transducer (2).

# FIG. 1

FIG. 2

(Z)

(Y)

(X)

2

1

P

S

F

K

FIG. 4

S

K

## FIG. 3